# FASCICULE DE BREVET EUROPEEN

(11) **EP 3 390 617 B1**
(45) Date de publication et mention de la délivrance du brevet: **06.09.2023**
(21) Numéro de dépôt: 16825486.0
(22) Date de dépôt: 13.12.2016
(51) Int. Cl.: C12N 1/12, C12P 7/64

(54) **PROCÉDÉ D'ENRICHISSEMENT DE PROTISTES EN LIPIDES RICHES EN ACIDES GRAS POLYINSATURÉS, PLUS PARTICULIÈREMENT DE CLASSE OMÉGA 3, ET SA MISE EN OEUVRE POUR LA PRODUCTION DE CES LIPIDES**
VERFAHREN ZUR ANREICHERUNG VON PROTISTEN MIT LIPIDEN, DIE REICH AN MEHRFACH UNGESÄTTIGTEN FETTSÄUREN SIND, INSBESONDERE DER OMEGA-3-KLASSE, UND VERWENDUNG DAVON ZUR HERSTELLUNG DER BESAGTEN LIPIDE
METHOD FOR ENRICHING PROTISTS WITH LIPIDS RICH IN POLYUNSATURATED FATTY ACIDS, MORE PARTICULARLY OF THE OMEGA 3 CLASS, AND IMPLEMENTATION OF SAME FOR THE PRODUCTION OF SAID LIPIDS

(30) Priorité: 14.12.2015 FR 1562310
(43) Date de publication de la demande: 24.10.2018
(73) Titulaire: Biorea, 22400 Lamballe-Armor (FR)
(72) Inventeur: ROMARI, Khadidja, 93400 Saint Ouen (FR)
(74) Mandataire: Germain Maureau
(86) Numéro de dépôt international: PCT/FR2016/053359
(87) Numéro de publication internationale: WO 2017/103421

(56) Documents cités:
- WO-A2-2013/010090
- WO-A2-2015/150716
- US-A1- 2011 165 658
- US-A1- 2013 217 085
- US-B2- 9 017 985
- GENNITY J M ET AL: "A selenite-induced decrease in the lipid content of a red alga", PHYTOCHEMISTRY, PERGAMON PRESS, GB, vol. 24, no. 12, 26 novembre 1985 (1985-11-26), pages 2823-2830, XP026609416, ISSN: 0031-9422, DOI: 10.1016/0031-9422(85)80007-8 [extrait le 1985-11-26]
- Andrew E Wheeler ET AL: "Elsevier Biomedical Press THE EFFECT OF SELENATE, SELENITE, AND SULFATE ON THE GROWTH OF SIX UNICELLULAR MARINE ALGAE", Mar. Biol. Ecol, 1 janvier 1982 (1982-01-01), pages 181-194, XP055272040, Extrait de l'Internet: URL:http://www.sciencedirect.com/science/a rticle/pii/0022098182901915/pdf?md5=48aa33 bbe3d6b53614258a355301345a&pid=1-s2.0-0022 098182901915-main.pdf [extrait le 2016-05-11]

## Description

L'invention concerne un procédé d'enrichissement de protistes, et en particulier de microalgues, en lipides riches en acides gras polyinsaturés, en particulier de classe oméga 3 (ω3), ledit procédé comprenant la mise en culture des protistes dans un milieu de culture comprenant au moins un composé sélénié, ainsi qu'un procédé de production de lipides riches en acides gras polyinsaturés, en particulier de classe w3, mettant en oeuvre le procédé d'enrichissement selon l'invention.

La présente invention a trait au domaine général de la production d'acides gras par des microorganismes, en particulier des protistes, tout particulièrement des microalgues.

Le terme « protistes » désigne des eucaryotes autres que les animaux, champignons et plantes. Ce groupe est très hétérogène et réunit des organismes à organisation cellulaire dite simple, unicellulaires (cas le plus fréquent) ou multicellulaires sans tissus spécialisés. Certains protistes sont autotrophes et d'autres sont hétérotrophes (comme les protozoaires se nourrissant de microalgues ou comme des microalgues de genre *Schizochytrium* ou de genre *Crypthecodinium*)*.*

Les acides gras polyinsaturés (AGPI) (ou PUFA pour « Poly Unsaturated Fatty Acid » en anglais) font partie des constituants principaux des lipides dans les systèmes biologiques. Un AGPI est formé d'une chaîne hydrocarbonée comportant au moins 18 atomes de carbone par molécule, terminée par un groupement carboxyle, et comporte au moins deux doubles liaisons carbone-carbone.

Les AGPI sont classés suivant leur nombre de carbone, leur nombre d'insaturations (ou doubles liaisons carbone-carbone) et l'emplacement de leur première insaturation en partant du groupement opposé au groupement carboxyle. Ainsi un acide gras polyinsaturé C X:Y ωZ est un acide gras comportant X atomes de carbone, Y insaturations et dont la première insaturation est en Zème position i.e. position n°Z, où X, Y et Z sont chacun un nombre entier, X étant supérieur ou égal à 18.

Les deux classes les plus importantes d'AGPI sont les classes des oméga 3 (w3) et oméga 6 (ω6).

Parmi les AGPI de classe w3 se trouvent l'acide α-linolénique (C 18:3 ω3), l'acide eicosapentaénoique (ou EPA) (C 20:5 w3) et l'acide docosahexaénoique (ou DHA) (C 22:6 ω3). Le DHA possède en tout six insaturations en positions 3, 6, 9, 12, 15 et 18.

D'une manière générale, les AGPI sont essentiels pour la croissance et la survie de tous les organismes dans le maintien de l'intégrité des membranes, dans le maintien de la perméabilité des membranes et dans le stockage cellulaire du carbone. Les mammifères qui ne possèdent pas les enzymes nécessaires à leur synthèse doivent donc les trouver dans leur nourriture.

Chez l'homme, les AGPI jouent un rôle indispensable dans le développement et le fonctionnement optimal du cerveau, du coeur et de bien d'autres organes et tissus. En particulier les AGPI de classe ω3 sont présentés comme les composés les plus bénéfiques sur la santé humaine à long terme, car ils diminuent les risques de maladies cardiovasculaires et les désordres immunologiques et inflammatoires. Ils agiraient également sur le système cérébral, le système hormonal et le système inflammatoire. Le DHA, notamment, permet un bon développement foetal et infantile au niveau visuel et neurologique, ce qui lui confère depuis quelques années un intérêt grandissant dans les différentes formulations des produits alimentaires, notamment des aliments pour nourrissons.

Ainsi, le marché des AGPI de classe w3 dépasse les 2 milliards de dollars en 2014. D'après l'étude de marché « Omega-3 PUF A Market by Type (DHA, EPA, ALA), Source [Marine (Fish, Algal, Krill, Others), Plant (Flaxseed, Chia Seed, Others)], Application (Dietary supplément, Functional F&B, Pharma, Infant Formula, Others) & Geography - Global Trend & Forecast to 2019 », ce marché dépasserait les 4 milliards de dollars en 2019. Certains aliments en sont naturellement riches comme les poissons gras (harengs, sardines, maquereaux...) et certaines huiles végétales (colza, noix, soja...). Cependant, si les huiles de poissons représentent actuellement la principale source industrielle d'AGPI, elles ne peuvent plus faire face durablement à l'augmentation de la demande, en particulier pour le DHA. En effet, la demande soutenue pour des huiles de poissons de haute qualité coïncide avec un contexte de surpêche, conduisant à l'instauration de réglementations sur la pêche de plus en plus strictes, et à une diminution de la production globale d'huile de poisson. La production d'huiles de poisson pose également des problèmes de raffinage, notamment à cause du goût et de l'odeur peu agréables de ces huiles de poisson, de leur faible stabilité oxydative et de leur teneur en cholestérol et en produits toxiques. De ce fait, leur utilisation a été limitée, notamment sur le marché des compléments alimentaires.

En conséquence, une source alternative commence à être utilisée pour ce marché florissant de la production d'AGPI : les microorganismes, notamment les microalgues. En effet, ces dernières, de par leur extraordinaire diversité et leur plasticité métabolique, représentent une source significative d'AGPI pour de nombreuses industries, notamment les industries alimentaire et pharmaceutique. Parmi ces microorganismes, se trouvent notamment des microalgues du règne des protistes telles que la famille des *Thraustochytrides* comprenant le genre *Schizochytrium* et la famille des *Dinoflagellata* (Dinoflagellés) comprenant le genre *Crypthecodinium.*

La composition en acides gras des microalgues est souvent plus simple que celle des huiles de poisson, réduisant le nombre d'étapes nécessaires pour concentrer les acides gras recherchés.

Les microalgues *Schizochytrium* et *Crypthecodinium* sont capables de produire des lipides en quantité allant jusqu'à 40-50 % de leur biomasse sèche et jusqu'à 30-40 % d'AGPI parmi les lipides totaux. Ces AGPI sont majoritairement stockés sous forme de triglycérides. En outre, ces microalgues marines ne présentent pas d'odeur désagréable puisqu'elles ne comprennent pas de cholestérol, et ne possèdent donc pas les inconvénients présentés par les huiles de poisson. Elles sont également bien perçues par le consommateur en tant que produit naturel d'origine végétale. Il est en outre possible de produire les microalgues à grande échelle dans des bioréacteurs en contrôlant tous les paramètres de culture. Par conséquent, les microalgues comptent désormais au premier plan des sources d'AGPI, en alternative aux huiles de poisson. Le DHA est en particulier utilisé dans les formulations pour nourrissons et prématurés, et dans les compléments alimentaires. Il peut être issu de l'aquaculture par l'utilisation des microalgues hétérotrophes marines *Crypthecodinium* et *Schizochytrium.*

Les conditions de culture des microalgues ont une grande influence sur les rendements en AGPI. Cependant, si les quantités de biomasse obtenues varient beaucoup, on observe que les proportions en EPA et DHA, par rapport à cette biomasse, restent globalement constantes. Une des voies explorées pour améliorer la croissance et la production des molécules à haute valeur ajoutée tels que les lipides et les caroténoïdes chez les microalgues est la supplémentation du milieu de culture de ces microalgues en composé(s) chimique(s).

Ainsi, la demande de brevet US 2013/0217085 enseigne que l'ajout de glycine bétaïne exogène ou de tréhalose au milieu de culture peut augmenter significativement le rendement de fermentation en acides gras polyinsaturés de la *Schizochytrium,* en particulier la *Schizochytrium limacinum;* l'ajout de n-dodécane (transporteur d'oxygène) dans le milieu de culture de *Crypthecodinium cohnii* a permis d'améliorer la production de DHA pour atteindre 51 % des lipides totaux, comme rapporté dans la publication Da Silva et al., « Effect of n-dodecane on Crypthecodinium cohnii fermentations and DHA production », J. Ind. Microbiol. Biotechnol. 33 pp 408-416, 2006.

Cependant, en dehors de l'image négative donnée au consommateur par l'utilisation de ces composés chimiques non biosourcés (c'est-à-dire n'étant pas d'origine végétale), l'ajout de ces composés, outre qu'il est coûteux, pose des problèmes de purification des microalgues puisqu'ils se retrouvent dans la biomasse finale.

D'autre part, la demande de brevet WO 2015/150716 décrit un procédé de culture de microalgues du genre *Aurantiochytrium,* en particulier un protiste du genre *Aurantiochytrium mangrovei,* en milieu de culture quasiment exempt de chlorure et quasiment exempt de sodium, pour la production de DHA. Les souches concernées sont des souches de *Thraustochytrides* du genre *Aurantiochytrium mangrovei* et *Aurantiochytrium limacinum.* Le sélénium est cité, parmi bien d'autres éléments, comme un véhicule du sodium pouvant éventuellement être présent en très faible quantité dans le milieu de culture (pas plus de 1 mg/L de sélénite de sodium soit pas plus de 0,46 mg/L de sélénium). En outre il est indiqué que les souches *Schizochytrium sp.* ne font expressément pas partie de l'invention.

Le document Gennity JM et al : « A selenite-induced decrease in the lipid content of a red alga » Phytochemistry, 1985, vol. 24, no. 12 page 2823-2830 concerne deux algues *Dunaliella primolecta* et *Porphyridium cruentum* qui cultivées en présence de sélénium possède une activité glutathione peroxydase. Les auteurs ont montré que dans ces conditions de culture la production d'acides gras polyinsaturés diminue.

Enfin, le document WO 2013/010090 décrit des compositions à haute teneur en lipides, ainsi que des procédés de fabrication de celles-ci. La teneur en lipides est d'au moins 67% par rapport à la matière sèche. L'algue est de préférence une souche ou une espèce du genre *Chlorella,* du genre *Schizochytrium* ou du genre *Crypthecodinium.* Quel que soit le milieu de culture, il contient des extraits de levure, de préférence de Saccharomyces cerevisiae, qui est de préférence enrichie en sélénium. Dans ce cas, le milieu de culture comprend un composé sélénié. La teneur en sélénium du milieu de culture est très élevée, à savoir de 10 à 100% pour 7,5 g/L de levure dans le milieu de culture, soit de 75 à 750 mg/L de sélénium.

Ainsi, il subsiste donc le besoin d'augmenter la production d'AGPI, en particulier de classe w3, par les microalgues, tout en simplifiant l'extraction à partir de la biomasse.

L'invention vise à pallier les inconvénients de l'état de la technique, en proposant une méthode d'augmentation de la production de lipides riches en acides gras polyinsaturés (AGPI), en particulier de classe ω3, tout particulièrement de DHA, par l'utilisation dans le milieu de culture d'un composé sélénié.

Par « riches en AGPI », on entend selon l'invention comprenant de 55% à 80%, de préférence de 60 à 75%, en poids (d'AGPI par rapport aux lipides totaux). En effet, de manière surprenante, le demandeur a constaté que l'ajout d'un composé sélénié dans le milieu de culture des protistes pouvait améliorer la production de lipides riches en acides gras polyinsaturés, en particulier de classe w3, tout particulièrement de DHA, tout en diminuant la quantité d'acides gras saturés présents dans ces lipides de la biomasse.

Un objet de l'invention est donc d'optimiser la production de lipides riches en AGPI, en particulier de classe ω3, plus particulièrement de DHA, par des protistes, de préférence par des microalgues, de façon encore plus préférée par des microalgues marines hétérotrophes, procurant une solution alternative aux solutions proposées par l'état de la technique, mais moins coûteuse et plus simple à mettre en oeuvre que celles-ci.

L'invention a ainsi pour objet, sous un premier aspect, un procédé d'enrichissement de protistes en lipides riches en acides gras polyinsaturés comprenant de 55% à 80% en poids d'acides gras polyinsaturés par rapport aux lipides totaux, en particulier et de préférence de classe oméga 3 (ω3), ledit procédé comprenant la mise en culture des protistes dans un milieu de culture comprenant au moins un composé sélénié,
la concentration dans le milieu de culture dudit composé sélénié étant telle que le sélénium est présent en une concentration comprise entre 1 et 8 mg/L.

Le composé sélénié est de préférence choisi dans le groupe formé par l'acide 2-hydroxy-4-méthylsélénobutyrique ou un de ses sels, le sélénite, l'acide sélénique, le sélénate, la sélénocystéine, la sélénométhionine, la sélénocystathionine, la sélénohomocystéine et la séléno-adénosylsélénomathionine.

Le composé sélénié est de façon encore plus préférée choisi dans le groupe formé par l'acide 2-hydroxy-4-méthylsélénobutyrique (ou acide 2-hydroxy-4- méthylsélénobutanoïque) ou un de ses sels, le sélénite, l'acide sélénique, et la sélénométhionine.

L'acide 2-hydroxy-4-méthylsélénobutyrique est généralement sous forme L, D, ou D, L.

Le sel de l'acide 2-hydroxy-4-méthylsélénobutyrique est de préférence un sel de calcium, de zinc ou de magnésium.

De façon particulièrement avantageuse selon l'invention, les lipides riches en acides gras polyinsaturés sont particulièrement riches en acide docosahexaénoique (DHA). Par « riche en DHA », on entend selon l'invention comprenant de 45% à 70%, de préférence de 48 à 60%, en poids (de DHA par rapport aux lipides totaux). Sauf indication contraire, tous les pourcentages sont donnés ici en % poids. Selon l'invention, les protistes sont de préférence des microalgues du règne des Chromalveolata.

Un des avantages de l'invention est que l'enrichissement en AGPI s'accompagne d'un appauvrissement (autrement dit d'une diminution) en acides gras saturés que produit le protiste à teneur totale en lipides identique ou supérieure par rapport au cas où, toutes choses égales par ailleurs, le milieu de culture ne comprend pas de composé sélénié. Cela signifie que, en comparaison avec les teneurs en acides gras saturés obtenues avec les protistes enrichis en AGPI selon l'art antérieur, le protiste comprend généralement une teneur réduite en acides gras saturés, c'est-à-dire généralement comprise dans un intervalle de 40 à 68%, par rapport aux lipides totaux.

Sans vouloir être liée par une quelconque théorie, la demanderesse pense que la diminution de la teneur des acides gras saturés se fait au bénéfice de la production des acides gras insaturés. Selon un premier mode de réalisation du procédé de production selon l'invention, ces microalgues sont de la division des *Stramenopiles,* de préférence de la famille des *Thraustochytrides* ou des *Labyrinthulides,* de façon encore plus préférée du genre *Schizochytrium, Ulkenia, Aurantiochytrium* ou *Thraustochytrium.* De préférence dans ce cas, ces microalgues sont de la division des *Stramenopiles,* de préférence de la famille des *Thraustochytrides,* de façon encore plus préférée du genre *Schizochytrium, Ulkenia* ou *Thraustochytrium.*

Selon un deuxième mode de réalisation du procédé de production selon l'invention, ces microalgues sont de la division des *Alveolata,* de préférence de la famille des *Dinoflagellata,* de façon encore plus préférée du genre *Crypthecodinium.*

Selon un mode de réalisation tout particulièrement préféré, les microalgues sont des microalgues marines hétérotrophes d'espèce *Schizochytrium limacinum* ou *Schizochytrium mangrovei,* ou des microalgues d'espèce *Crypthecodinium cohnii,* de préférence des microalgues marines hétérotrophes d'espèce *Schizochytrium limacinum,* ou des microalgues d'espèce *Crypthecodinium cohnii.* Mais des microalgues hétérotrophes d'espèce *Crypthecodinium semeuse, Schizochytrium aggregatum, Ulkenia profunda, Ulkenia radiata, Ulkenia visurgensis, Aurantiochytrium mangrovei, Aurantiochytrium limacinum, Thraustochytrium globosum, Thraustochytrium aureum, Thraustochytrium pachyderma, Thraustochytrium aggregatum* et/ou *Thraustochytrium striatum,* de préférence d'espèce *Crypthecodinium semeuse, Schizochytrium aggregatum, Ulkenia profunda, Ulkenia radiata, Ulkenia visurgensis, Thraustochytrium aureum, Thraustochytrium pachyderma, Thraustochytrium aggregatum* et/ou *Thraustochytrium striatum,* peuvent également être utilisées dans le cadre de l'invention.

Par « A et/ou B », on entend selon l'invention A, ou B, ou A et B.

En dehors de la présence du ou des composé(s) sélénié(s) selon l'invention, le milieu de culture est tel que connu de l'homme du métier, en particulier pour l'espèce concernée, c'est-à-dire qu'il comporte un milieu de nutrition dans les éléments chimiques nécessaires à la croissance du protiste (azote ; sels minéraux ; carbone ; etc .).

Dans le cas particulier de l'acide 2-hydroxy-4-méthylsélénobutyrique, on peut se référer pour les conditions d'utilisation de cet acide au brevet US9017985 de la demanderesse, qui concerne l'utilisation de cet acide pour l'enrichissement en sélénium d'un microorganisme photosynthétique.

Le procédé d'enrichissement selon l'invention est tel que la concentration dans le milieu de culture dudit composé sélénié est telle que le sélénium est présent en une concentration comprise entre 1 et 8 mg/L, par exemple de 5 mg/L.

Lorsque plusieurs composés séléniés sont présents, cela vaut pour la teneur totale en sélénium du milieu, prenant en considération chacun d'entre eux.

Le procédé d'enrichissement est généralement effectué sur une durée de 96 h à 200 h, par exemple sur une durée égale à 187 h.

L'invention concerne également les protistes enrichis en lipides riches en acides gras polyinsaturés, en particulier de classe oméga 3 (ω3), obtenus selon le procédé d'enrichissement de l'invention, les protistes étant des microalgues marines hétérotrophes.

De façon particulièrement avantageuse selon l'invention, ces protistes sont également enrichis en sélénium, c'est-à-dire qu'ils comprennent entre 5 et 800 ppm (en poids) de sélénium par rapport à leur masse totale.

Les protistes enrichis en lipides riches en AGPI, en particulier de classe ω3, obtenus par le procédé d'enrichissement selon l'invention sont également utiles en tant qu'agent cosmétique ou nutritionnel. Cette masse séléniée enrichie en lipides riches en AGPI, en particulier de classe ω3, peut avantageusement être valorisée, tout particulièrement en nutrition humaine ou animale, de préférence animale.

L'invention concerne donc également l'utilisation de ces protistes enrichis en lipides riches en AGPI, en particulier de classe ω3, obtenus par le procédé d'enrichissement selon l'invention, en nutrition ou cosmétique. De façon corollaire, l'invention concerne tout produit alimentaire ou cosmétique comprenant les protistes enrichis en lipides riches en AGPI, en particulier de classe ω3, obtenus par le procédé d'enrichissement selon l'invention.

Ainsi, les protistes enrichis en lipides riches en AGPI, en particulier de classe ω3, obtenus par le procédé d'enrichissement selon l'invention peuvent tout particulièrement être utilisés en nutrition humaine ou animale.

Cependant, une telle utilisation peut également être effectuée après traitement d'extraction des lipides riches en AGPI de la biomasse voire d'extraction des lipides riches en AGPI de la biomasse et de purification des lipides ainsi extraits.

Cette autre utilisation est d'autant plus intéressante qu'un autre des avantages de l'invention est que, de façon surprenante, lesdits lipides sont également enrichis en sélénium, c'est-à-dire qu'ils comprennent plus de 1 ppm, de façon encore plus préférée plus de 2 ppm (en poids) de sélénium par rapport à leur masse totale. Dans tous les cas ils comprennent généralement moins de 30 ppm de sélénium par rapport à leur masse totale. La présence à cette teneur relativement élevée de sélénium dans les lipides est une autre caractéristique du procédé selon l'invention. On peut parler ainsi de « lipides microalgaux séléniés » ou « lipides séléniés ».

L'extraction peut être réalisée par toute méthode d'extraction connue de l'homme du métier telle qu'une extraction par au moins un solvant organique ou une extraction par du dioxyde de carbone à l'état supercritique.

Ainsi, l'invention a pour objet, sous un second aspect, un procédé de production de lipides riches en acides gras polyinsaturés, comprenant de 55% à 80% en poids d'acides gras polyinsaturés, en particulier de classe oméga 3 (ω3), comprenant successivement :
- un procédé d'enrichissement de protistes en lipides riches en acides gras polyinsaturés, en particulier de classe ω3, selon l'invention, de façon à obtenir des protistes enrichis en lipides riches en acides gras polyinsaturés comprenant de 55% à 80% en poids d'acides gras polyinsaturés, en particulier de classe ω3; et
- un procédé de traitement des protistes enrichis en lipides riches en acides gras polyinsaturés comprenant de 55% à 80% en poids d'acides gras polyinsaturés, en particulier de classe ω3, par extraction de la biomasse des lipides riches en acides gras polyinsaturés comprenant de 55% à 80% en poids d'acides gras polyinsaturés, en particulier de classe ω3, de façon à obtenir lesdits lipides (séléniés) riches en acides gras polyinsaturés, en particulier de classe ω3, et une biomasse séléniée appauvrie en lipides.

La biomasse séléniée appauvrie en lipides riches en AGPI, obtenue après cette extraction, peut également être avantageusement utilisée, entre autres en nutrition généralement humaine ou animale, de préférence animale.

Le procédé de traitement des protistes enrichis en lipides riches en acides gras polyinsaturés, en particulier de classe ω3, consiste donc en une extraction de la biomasse des lipides riches en acides gras polyinsaturés, le plus souvent et de préférence suivie d'une purification des lipides ainsi extraits (séparés de la biomasse) riches en acides gras polyinsaturés, en particulier de classe ω3. Ce procédé de traitement est en général suivi d'une analyse du profil des lipides ainsi obtenus.

La méthode d'extraction des lipides de la biomasse est connue de l'homme du métier et est, par exemple, précisée dans l'exemple ci-après. L'invention a ainsi pour objet, sous un troisième aspect, les lipides riches en acides gras polyinsaturés, en particulier de classe oméga 3 (ω3), obtenus selon le procédé de production de l'invention qui sont, avantageusement, également enrichis en sélénium et à ce titre qualifiés de « lipides séléniés ».

Les lipides peuvent être utilisés tels quels, le plus souvent en nutrition humaine ou animale.

Ainsi le procédé de production de lipides riches en acides gras polyinsaturés, en particulier de classe ω3, selon l'invention, comprend la mise en oeuvre du procédé d'enrichissement selon l'invention, ce procédé d'enrichissement possédant les mêmes caractéristiques que celles explicitées ci-dessus.

L'invention concerne enfin tout produit alimentaire ou cosmétique comprenant des lipides riches en acides gras polyinsaturés, en particulier de classe oméga 3 (ω3), obtenus par le procédé de production selon l'invention. L'invention sera mieux comprise à la vue des exemples de réalisation qui suivent, en référence aux dessins annexés dans lesquels :
- la Figure 1 est un graphe donnant le pourcentage poids des acides gras polyinsaturés (AGPI) dans les lipides totaux après 187 h d'incubation pour la *Schizochytrium limacinum ;*
- la Figure 2 est un graphe donnant le pourcentage poids d'acide docosahexaénoique (DHA) dans les lipides totaux après 187 h d'incubation pour la *Schizochytrium limacinum ;*
- la Figure 3 est un graphe donnant le pourcentage poids des acides gras saturés (AGS) dans les lipides totaux après 187 h d'incubation pour la *Schizochytrium limacinum* ; et
- la Figure 4 est un graphe donnant le pourcentage poids des acides gras polyinsaturés (AGPI) dans les lipides totaux, le pourcentage poids de l'acide docosahexaénoique (DHA) dans les lipides totaux et le pourcentage poids des acides gras saturés (AGS) dans les lipides totaux, après 187 h d'incubation pour le *Crypthecodinium cohnii.*

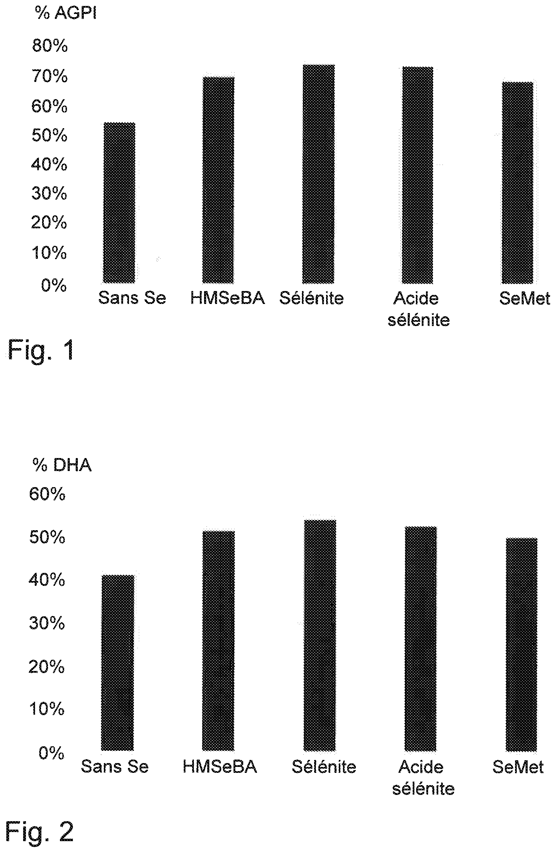

Les Figures 1 à 4 sont explicitées dans les exemples 1 et 2 ci-après.

### EXEMPLES

Les exemples qui suivent illustrent l'invention sans pour autant en limiter la portée.

### EXEMPLE 1 . Culture du protiste Schizochytrium dans un milieu contenant séparément de l'acide

### 2-hvdroxy-4-méthylsélénobutyrique (HMSeBA) ; sélénite ; de l'acide sélénique ; ou de la sélénométhionine

Cette culture a conduit à l'amélioration de la production des acides gras polyinsaturés et de DHA en particulier.

La *Schizochytrium* est un protiste, et plus précisément une micro-algue Thraustochytride hétérotrophe.

Une souche de *Schizochytrium limacinum* ATCC- MYA1381 a été obtenue de la banque ATCC (www.atcc.org).

Les caractéristiques de croissance ainsi que la production des lipides de *Schizochytrium limacinum* ont été mesurées en présence de différentes formes de sélénium et comparées en l'absence de tels additifs (contrôle référencé « Sans Se » sur les Figures).

La pré-culture de la souche *Schizochytrium limacinum* ATCC- MYA1381 a été réalisée en milieu liquide standard, dont la composition est donnée dans le Tableau 1. Pour ce faire, des cultures de démarrage (ou « starter ») de 50 mL ont été lancées en récipient Erlen de 250 mL à partir d'une colonie isolée sur 633 M3 chytrid agar (www.atcc.org) et mises en culture à 25°C ± 1 °C en hétérotrophie pendant 48 h dans ce milieu liquide standard. L'inoculum de 100 mL est ensuite lancé en récipient Erlen de 500 mL à partir de ces cultures « starter » à une concentration initiale de 1 ,0.10⁶cellules/mL dans les mêmes conditions de culture que ce dernier durant 4 jours. Les cultures ont été aérées en continu par agitation orbitale de 100 rpm (pour « tour par minute »). Les cultures d'enrichissement en composé sélénié ont été lancées à partir de l'inoculum à une concentration initiale de 1 ,0.10⁶cellules/mL pour un volume final de 400 mL en récipient Erlen de 2,0 L à 25°C ± 1 °C en hétérotrophie pendant 187 h. Les cultures sont aérées en continu par agitation orbitale de 100 rpm. L'enrichissement a été effectué à TO par ajout de solution concentrée en sélénium (solution d'acide 2-hydroxy-4-méthylsélénobutyrique vendue par la société Tetrahedron à 0,0125 g/L référencée « HMSeBA » sur les Figures 1 à 3, sélénite de sodium 0,0110 g/L référencé « Sélénite » sur les Figures 1 à 3, acide sélénique 0,0082 g/L référencé « Acide sélénique » sur les Figures 1 à 3 et sélénométhionine 0,0124 g/L référencée « SeMet » sur les Figures 1 à 3) pour une concentration finale de 5 mg de sélénium/L dans le milieu, quelle que soit la source de composé sélénié.

La biomasse de *Schizochytrium limacinum* a ensuite été récoltée après 72 h et 187 h d'incubation.

**Tableau 1. Résumé des concentrations de chaque réactif composant le milieu liquide standard de culture**

| **Réactifs** | **Concentration finale (g/L)** |
|---|---|
| Eau de mer artificielle | 33 |
| Extrait de levure | 8,7 |
| Glucose monohydrate (G8270 Sigma) | 17,0 |

L'eau de mer artificielle est un produit courant du commerce, dans ce cas c'était le produit « Sel Instant Ocean » de la société AQUARIUM SYSTEMS. Sa composition est donnée ci-après à titre informatif : Na⁺ (0.35 g/L) ; K⁺ (0.01 g/L) ; Mg²⁺ (0.04 g/L); Ca²⁺ (0.01 g/L) ; Sr' (0.00054 g/L) ; Cl (0.641 g/L); S (SO4²⁺) (0.07 g/L); P (PO₄) (0.000157 mg/L); N (NO3) (0.002 mg/L); N (NH4) (0.006 mg/L); Si (SiO3) (0.0105 mg/L); Li (0.0124 mg/L); Si (0.0148 mg/L); Mo (0.0057 mg/L); Ba (0.00385 mg/L); V (0.0048 mg/L); Ni (0.00329 mg/L); Cr (0.0129 mg/L); Al (0.214 mg/L); Cu (0.00377 mg/L); Zn (0.00108 mg/L); Mn (0.00218 mg/L); Fe (0.000442 mg/L); Cd (0.000890 mg/L); Pb (0.0144 mg/L); Co (0.00253 mg/L); Ag (0.00819 mg/L) : Ti (0.00106 mg/L).

L'extrait de levure est un produit commercial standard fourni par la société Merck.

### Suivi des cultures

La concentration en biomasse totale a été suivie par mesure de la masse sèche (filtration sur filtre GFC, Whatman, puis séchage à l'étuve sous vide, 70°C et -0,8 bar (80 kPa), pendant 24h minimum avant pesée).

10⁷ cellules/mL ont été extraites pour quantifier les lipides totaux. La méthode d'extraction des lipides est connue de l'homme du métier et est telle que décrite par la publication « Bligh E.G. & Dyer W.J., A rapid method of total lipid extraction and purification, Can. J. Biochem. Physiol 37 1959 91 1-917 », cette méthode ayant été légèrement adaptée pour les cellules de microalgue. Ainsi, pour chaque mesure, environ 10 mL de culture (biomasse et milieu) fraîchement prélevée a été lavée avec de l'eau déminéralisée de façon à éliminer les sels extracellulaires avant lyophilisation, puis placée dans un tube en verre et centrifugée pendant 10 min à 3600g et à 4°C. Après centrifugation, le surnageant a été éliminé et le culot contenant les cellules a été lyophilisé. L'extraction a été réalisée avec 6 mL d'un mélange monophasique CHCIs/MeOH (chloroforme/méthanol) dans les proportions 2/1 (VA/). Pour assurer une extraction complète des lipides, le tube a été maintenu en agitation sur un balancier pendant 6 h à l'obscurité, puis stocké à -20°C avant d'être analysé par GC-FID (chromatographie en phase gazeuse avec détection par ionisation de flamme) pour déterminer la quantité et les profils des acides gras totaux extraits.

Les résultats obtenus sont illustrés par les Figures 1, 2 et 3 qui donnent respectivement, pour la *Schizochytrium limacinum,* après 187 h d'incubation, le pourcentage d'AGPl dans les lipides totaux, le pourcentage de DHA dans les lipides totaux et le pourcentage d'AGS dans les lipides totaux en ordonnée, pour chacune des sources séléniées indiquée en abscisse.

On a constaté que la présence de composé sélénié à 5 mg/L dans le milieu de culture a permis d'obtenir un pourcentage d'AGPI nettement supérieur à celui du contrôle (au minimum 67% contre 53%), quelle que soit l'espèce séléniée utilisée, le résultat le plus intéressant étant donné par le sélénite (73%). On a également constaté que la présence de composé sélénié à 5 mg/L dans le milieu de culture a permis d'obtenir un pourcentage de DHA nettement supérieur à celui du contrôle (au minimum 50% contre 41 %), quelle que soit l'espèce séléniée utilisée, le résultat le plus intéressant étant donné par le sélénite (54%).

On a enfin constaté que la présence de composé sélénié à 5 mg/L dans le milieu de culture a permis d'obtenir un pourcentage d'AGS nettement inférieur à celui du contrôle (au minimum 31 % contre 44%), quelle que soit l'espèce séléniée utilisée, le résultat le plus intéressant étant donné par le sélénite (26%).

### EXEMPLE 2. Culture du protiste Crypthecodinium cohnii dans un milieu contenant de la sélénométhionine

Cette culture a conduit à l'amélioration de la production des acides gras polyinsaturés et de DHA en particulier.

Il a été procédé de la même manière qu'à l'exemple 1, à l'exception du fait que le protiste est le *Crypthecodinium cohnii* au lieu de la *Schizochytrium limacinum,* et qu'une seule source de sélénium a été utilisée : la sélénométhionine (« SeMet »).

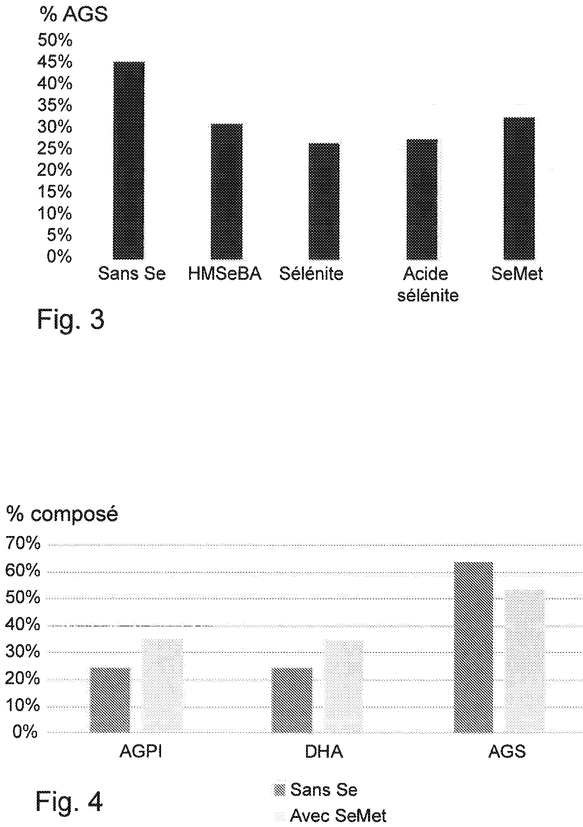

Le *Crypthecodinium cohnii* est un protiste, et plus précisément une micro-algue hétérotrophe. La souche *Crypthecodinium cohnii* CCMP 316 a été obtenue de la banque NCMA (https://ncma.bigelow.org). Les résultats obtenus sont illustrés par la Figure 4 qui donne, pour le *Crypthecodinium cohnii,* après 187 h d'incubation, le pourcentage de composé dans les lipides totaux en ordonnée, pour chacun des composés (AGPI, DHA et AGS) indiqué en abscisse, en présence de sélénométhionine (référence « Avec SeMet »), ou en l'absence de composé sélénié (référence « Sans Se »). On a constaté que la présence de sélénométhionine à 5 mg/L de sélénium dans le milieu de culture a permis d'obtenir un pourcentage d'AGPI nettement supérieur à celui du contrôle (35% contre 25%).

On a également constaté que la présence de sélénométhionine à 5 mg/L de sélénium dans le milieu de culture a permis d'obtenir un pourcentage de DHA nettement supérieur à celui du contrôle (34% contre 24%).

On a enfin constaté que la présence de sélénométhionine à 5 mg/L de sélénium dans le milieu de culture a permis d'obtenir un pourcentage d'AGS nettement inférieur à celui du contrôle (53,5% contre 64%).

Par ailleurs, on a constaté une augmentation de la teneur en lipides totaux dans la biomasse sèche grâce à l'utilisation de la sélénométhionine. En effet, on obtient une quantité de 34% de plus de lipides totaux en utilisant la culture en milieu sélénié par rapport au milieu de contrôle.

## Revendications

1. Procédé d'enrichissement de protistes en lipides comprenant de 55% à 80% en poids d'acides gras polyinsaturés par rapport aux lipides totaux,
ledit procédé comprenant la mise en culture des protistes dans un milieu de culture comprenant au moins un composé sélénié,
la concentration dudit composé sélénié dans le milieu de culture étant telle que la concentration de sélénium dans le milieu de culture est comprise entre 1 et 8 mg/L.

2. Procédé selon la revendication 1, dans lequel le composé sélénié est choisi dans le groupe formé par l'acide 2-hydroxy-4-méthylsélénobutyrique ou un de ses sels, le sélénite, l'acide sélénique, le sélénate, la sélénocystéine, la sélénométhionine, la sélénocystathionine, la sélénohomocystéine et la séléno-adénosylsélénomathionine.

3. Procédé selon l'une des revendications 1 ou 2, dans lequel le composé sélénié est choisi l'acide 2-hydroxy-4-méthylsélénobutyrique ou un de ses sels, le sélénite, l'acide sélénique, et la sélénométhionine.

4. Procédé selon l'une des revendications 1 à 3, dans lequel les microalgues sont de la division des *Stramenopiles,* de préférence de la famille des *Thraustochytrides* ou des *Labyrinthulides,* de façon encore plus préférée du genre *Schizochytrium, Ulkenia, Aurantiochytrium* ou *Thraustochytrium.*

5. Procédé selon l'une des revendications 1 à 3, dans lequel les microalgues sont de la division des *Alveolata,* de préférence de la famille des *Dinoflagellata,* de façon encore plus préférée du genre *Crypthecodinium.*

6. Procédé selon l'une des revendications 1 à 3, dans lequel les microalgues sont des microalgues marines hétérotrophes d'espèce *Schizochytrium limacinum* ou *Schizochytrium mangrovei,* ou des microalgues d'espèce *Crypthecodinium cohnii.*

7. Procédé selon l'une des revendications 5 ou 6, dans lequel les microalgues hétérotrophes sont des microalgues d'espèce *Schizochytrium limacinum* ou des microalgues d'espèce *Crypthecodinium cohnii.*

8. Protistes obtenus par le procédé d'enrichissement en lipides comprenant de 55% à 80% en poids d'acides gras polyinsaturés, en particulier de classe oméga 3, par rapport aux lipides totaux, selon l'une des revendications 1 à 7, les protistes étant des microalgues marines hétérotrophes.

9. Produit alimentaire ou cosmétique comprenant des protistes selon la revendication 8.

10. Procédé de production de lipides comprenant de 55% à 80% en poids d'acides gras polyinsaturés, par rapport aux lipides totaux, ledit procédé comprenant successivement :
- un procédé d'enrichissement de protistes selon l'une des revendications 1 à 7, de façon à obtenir des protistes enrichis en lipides comprenant de 55% à 80% en poids d'acides gras polyinsaturés par rapport aux lipides totaux, et
- un procédé de traitement des protistes enrichis par extraction de la biomasse des lipides comprenant de 55% à 80% en poids d'acides gras polyinsaturés, par rapport aux lipides totaux, de façon à obtenir des lipides comprenant de 55% à 80% en poids d'acides gras polyinsaturés, par rapport aux lipides totaux, et une biomasse séléniée appauvrie en lipides.

11. Procédé de production de lipides comprenant de 55% à 80% en poids d'acides gras polyinsaturés, par rapport aux lipides totaux, selon la revendication 10, dans lequel l'extraction est suivie d'une purification des lipides ainsi extraits comprenant de 55% à 80% en poids d'acides gras polyinsaturés, par rapport aux lipides totaux.

12. Lipides obtenus par le procédé selon l'une des revendications 10 ou 11, lesdits lipides comprenant de 55% à 80% en poids d'acides gras polyinsaturés par rapport aux lipides totaux, et plus de 1 ppm en poids de sélénium par rapport à leur masse totale.

13. Lipides selon la revendication 12 tels qu'ils comprennent plus de 2 ppm, en poids, de sélénium par rapport à leur masse totale.

14. Produit alimentaire ou cosmétique comprenant les lipides séléniés selon la revendication 12 ou 13.

15. Produit alimentaire selon la revendication 14, pour la nutrition humaine ou animale.

## Patentansprüche

1. Verfahren zur Anreicherung von Protisten mit Lipiden, die bezogen auf die Gesamtlipide 55 Gew.-% bis 80 Gew.-% mehrfach ungesättigte Fettsäuren umfassen,
wobei das Verfahren das Kultivieren der Protisten in einem Kulturmedium umfasst, das mindestens eine Selenverbindung umfasst,
wobei die Konzentration der Selenverbindung in dem Kulturmedium so ist, dass die Selenkonzentration in dem Kulturmedium zwischen 1 und 8 mg/L liegt.

2. Verfahren nach Anspruch 1, wobei die Selenverbindung ausgewählt ist aus der Gruppe bestehend aus 2-Hydroxy-4-methylselenobuttersäure oder einem ihrer Salze, Selenit, Selensäure, Selenat, Selenocystein, Selenomethionin, Selenocystathionin, Selenohomocystein und Selenoadenosylselenomathionin.

3. Verfahren nach einem der Ansprüche 1 oder 2, wobei die Selenverbindung ausgewählt ist aus 2-Hydroxy-4-methylselenobuttersäure oder einem ihrer Salze, Selenit, Selensäure und Selenomethionin.

4. Verfahren nach einem der Ansprüche 1 bis 3, wobei die Mikroalgen aus der Abteilung der *Stramenopiles,* vorzugsweise aus der Familie der *Thraustochytriden* oder der *Labyrinthuliden,* noch bevorzugter aus der Gattung *Schizochytrium, Ulkenia, Aurantiochytrium* oder *Thraustochytrium* stammen.

5. Verfahren nach einem der Ansprüche 1 bis 3, wobei die Mikroalgen aus der Division der *Alveolata,* vorzugsweise aus der Familie der *Dinoflagellata,* noch bevorzugter aus der Gattung *Crypthecodinium* stammen.

6. Verfahren nach einem der Ansprüche 1 bis 3, wobei es sich bei den Mikroalgen um heterotrophe marine Mikroalgen der Art *Schizochytrium limacinum* oder *Schizochytrium mangrovei* oder Mikroalgen der Art *Crypthecodinium cohnii* handelt.

7. Verfahren nach einem der Ansprüche 5 oder 6, wobei es sich bei den heterotrophen Mikroalgen um Mikroalgen der Spezies *Schizochytrium limacinum* oder Mikroalgen der Spezies *Crypthecodinium cohnii* handelt.

8. Durch das Lipidanreicherungsverfahren erhaltene Protisten, die bezogen auf die Gesamtlipide 55 Gew.-% bis 80 Gew.-% mehrfach ungesättigte Fettsäuren umfassen, insbesondere der Omega-3-Klasse, nach einem der Ansprüche 1 bis 7, wobei es sich bei den Protisten um heterotrophe marine Mikroalgen handelt.

9. Lebensmittel- oder Kosmetikprodukt, das Protisten nach Anspruch 8 umfasst.

10. Verfahren zur Herstellung von Lipiden, die bezogen auf die Gesamtlipide 55 Gew.-% bis 80 Gew.-% mehrfach ungesättigte Fettsäuren umfassen, wobei das Verfahren nacheinander Folgendes umfasst:
- Ein Verfahren zur Anreicherung von Protisten nach einem der Ansprüche 1 bis 7, solcherart, dass man mit Lipiden angereicherte Protisten erhält, die bezogen auf die Gesamtlipide 55 Gew.-% bis 80 Gew.-% mehrfach ungesättigte Fettsäuren umfassen; und
- ein Verfahren zur Behandlung der angereicherten Protisten durch Extraktion der Biomasse aus Lipiden, die bezogen auf die Gesamtlipide 55 Gew.-% bis 80 Gew.-% mehrfach ungesättigte Fettsäuren umfassen, um Lipide, die bezogen auf die Gesamtlipide 55 Gew.-% bis 80 Gew.-% mehrfach ungesättigte Fettsäuren umfassen, und eine an Lipiden verarmte selenhaltige Biomasse zu erhalten.

11. Verfahren zur Herstellung von Lipiden, die bezogen auf die Gesamtlipide 55 Gew.-% bis 80 Gew.-% mehrfach ungesättigte Fettsäuren umfassen, nach Anspruch 10, wobei auf die Extraktion eine Reinigung der so extrahierten Lipide folgt, die bezogen auf die Gesamtlipide 55 Gew.-% bis 80 Gew.-% mehrfach ungesättigte Fettsäuren umfassen.

12. Lipide, die durch das Verfahren nach einem der Ansprüche 10 oder 11 erhalten wurden, wobei die Lipide bezogen auf die Gesamtlipide 55 Gew.-% bis 80 Gew.-% mehrfach ungesättigte Fettsäuren und bezogen auf die Gesamtmasse mehr als 1 ppm Selen umfassen.

13. Lipide nach Anspruch 12, die bezogen auf die Gesamtmasse mehr als 2 Gew.-ppm Selen umfassen.

14. Lebensmittel- oder Kosmetikprodukt, das die selenhaltigen Lipide nach Anspruch 12 oder 13 umfasst.

15. Lebensmittelprodukt nach Anspruch 14 für die menschliche oder tierische Ernährung.

## Claims

1. A method for enriching protists with lipids comprising from 55% to 80% by weight of polyunsaturated fatty acids relative to the total lipids, said method comprising the culturing of the protists in a culture medium comprising at least one selenium compound, the concentration of said selenium compound in the culture medium being such that the concentration of selenium in the culture medium is comprised between 1 and 8 mg/L.

2. The method according to claim 1, wherein the selenium compound is selected from the group formed by 2-hydroxy-4-methylselenobutyric acid or one of its salts, selenite, selenic acid, selenate, selenocysteine, selenomethionine, selenocystathionine, selenohomocysteine and seleno-adenosylselenomathionine.

3. The method according to any of claims 1 or 2, wherein the selenium compound is selected from 2-hydroxy-4-methylselenobutyric acid or one of its salts, selenite, selenic acid, and selenomethionine.

4. The method according to any of claims 1 to 3, wherein the microalgae are from the *Stramenopiles* division, preferably from the *Thraustochytrid* or *Labyrinthulid* family, even more preferably from the *Schizochytrium, Ulkenia, Aurantiochytrium* or *Thraustochytrium* genus.

5. The method according to any of claims 1 to 3, wherein the microalgae are of the *Alveolata* division, preferably of the *Dinoflagellata* family, even more preferably of the *Crypthecodinium* genus.

6. The method according to any of claims 1 to 3, wherein the microalgae are heterotrophic marine microalgae of the *Schizochytrium limacinum* or *Schizochytrium mangrovei* species, or microalgae of the *Crypthecodinium cohnii* species.

7. The method according to any of claims 5 or 6, wherein the heterotrophic microalgae are microalgae of the *Schizochytrium limacinum* species or microalgae of the *Crypthecodinium cohnii* species.

8. A protists obtained by the lipid enrichment method comprising from 55% to 80% by weight of polyunsaturated fatty acids, in particular of the omega 3 class, relative to the total lipids, according to any of claims 1 to 7, the protists being heterotrophic marine microalgae.

9. A food or cosmetic product comprising protists according to claim 8.

10. A method for producing lipids comprising from 55% to 80% by weight of polyunsaturated fatty acids, relative to the total lipids, said method successively comprising:
- a method for enriching protists according to any of claims 1 to 7, so as to obtain protists enriched in lipids comprising from 55% to 80% by weight of polyunsaturated fatty acids relative to the total lipids; and
- a method for treating protists enriched by extraction from the biomass of lipids comprising from 55% to 80% by weight of polyunsaturated fatty acids, relative to the total lipids, so as to obtain lipids comprising from 55% to 80% by weight of polyunsaturated fatty acids, relative to total lipids, and a selenium biomass depleted in lipids.

11. The method for producing lipids comprising from 55% to 80% by weight of polyunsaturated fatty acids, relative to the total lipids, according to claim 10, in which the extraction is followed by a purification of the lipids thus extracted comprising 55% to 80% by weight of polyunsaturated fatty acids, based on total lipids.

12. A lipids obtained by the method according to any of claims 10 or 11, said lipids comprising from 55% to 80% by weight of polyunsaturated fatty acids relative to the total lipids, and more than 1 ppm by weight of selenium relative to their total mass.

13. The lipids according to claim 12 such that they comprise more than 2 ppm, by weight, of selenium relative to their total mass.

14. A food or cosmetic product comprising the selenium lipids according to claim 12 or 13.

15. The food product according to claim 14, for human or animal nutrition.
